(19) Europäisches Patentamt
European Patent Office
Office européen des brevets

(11) **EP 3 081 211 A1**

(12) **EUROPEAN PATENT APPLICATION**
published in accordance with Art. 153(4) EPC

(43) Date of publication:
**19.10.2016 Bulletin 2016/42**

(21) Application number: **14869001.9**

(22) Date of filing: **10.12.2014**

(51) Int Cl.:
*A61K 9/70* (2006.01)     *A61K 31/27* (2006.01)
*A61K 47/32* (2006.01)     *A61K 47/34* (2006.01)
*A61P 25/28* (2006.01)

(86) International application number:
**PCT/JP2014/082727**

(87) International publication number:
**WO 2015/087927 (18.06.2015 Gazette 2015/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.12.2013 JP 2013257427**
**24.12.2013 JP 2013265780**

(71) Applicant: **Hisamitsu Pharmaceutical Co., Inc.**
**Tosu-shi, Saga 841-0017 (JP)**

(72) Inventors:
• **SHINODA, Tomohiro**
  **Tsukuba-shi**
  **Ibaraki 305-0856 (JP)**
• **MICHINAKA, Yasunari**
  **Tsukuba-shi**
  **Ibaraki 305-0856 (JP)**

(74) Representative: **Grünecker Patent- und Rechtsanwälte**
**PartG mbB**
**Leopoldstraße 4**
**80802 München (DE)**

(54) **PATCH WITH COVER MEMBER AND PATCH KIT WITH COVER MEMBER**

(57) A cover material-equipped patch comprising:
a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa - SPb| \leq 1.5 \cdots (1),$$

$$2.0 \leq (SPa - SPc) \leq 2.8 \cdots (2),$$

and

$$1.0 \leq (SPb - SPc) \leq 2.8 \cdots (3).$$

Fig.1

**Description**

[Technical Field]

The present invention relates to a cover material-equipped patch and a cover material-equipped patch kit.

[Background Art]

Transdermally/transmucosally absorbable preparations have been developed as pharmaceutical preparations for administration of drugs into living organisms. Especially, patches have attracted attention, which are easy to handle and which enable an effective blood concentration of a drug to be kept for a long period. For example, drugs for Alzheimer-type dementia such as rivastigmine (3-[(S)-1-(dimethylamino)ethyl]phenyl methylethylcarbamate) are preferably administered in the form of patch from the viewpoints that loads on the caregivers to manage the administration of the drugs can be reduced, and that the drugs can be administered continuously. As the patches, for example, International Application Japanese-Phase Publication No. 2009-517468 (PTL 1) describes a transdermal therapeutic system comprising: a reservoir layer comprising a pharmaceutically active ingredient and a polymer; and an adhesive layer comprising a silicone polymer and a tackifier, and Japanese Patent No. 5093545 (PTL 2) describes a patch for treatment of Alzheimer's disease comprising a backing, a rivastigmine-containing layer, a pressure-sensitive adhesive layer, and a release liner. However, each of the patches is still insufficient in terms of the adhesion to the skin, and has such a problem that it is difficult to keep the patch attached to the skin for a long period (preferably for 3 to 7 days or longer).

Meanwhile, a cover material which is attached to the skin while covering a patch to prevent the patch from peeling off has been known from the past. As the cover material-equipped patch, for example, International Publication No. WO2011/074636 (PTL 3) describes a percutaneous absorption preparation comprising: a layered product comprising a drug-containing layer and a support layer; and a fixation means which can fix the layered product on a skin, International Publication No. WO2005/07266 (PTL 4) describes a cover material-equipped patch comprising: a cover material comprising a pressure-sensitive adhesive agent layer on one surface of a support; and a patch comprising a drug-containing layer on one surface of a support film, wherein the cover material and the patch are attached to each other, and Japanese Unexamined Patent Application Publication No. Sho 61-158924 (PTL5) describes a transdermal therapeutic system in which a skin impermeable backing and an adhesive overlay are formed on a drug/permeation enhancer reservoir.

[Citation List]

[Patent Literature]

[PTL 1] International Application Japanese-Phase Publication No. 2009-517468
[PTL 2] Japanese Patent No. 5093545
[PTL 3] International Publication No. WO2011/074636
[PTL 4] International Publication No. WO2005/072669
[PTL 5] Japanese Unexamined Patent Application Publication No. Sho 61-158924

[Summary of Invention]

[Technical Problem]

However, the present inventors have found that, when a patch and a cover material are stacked on each other as in a conventional cover material-equipped patch, the drug is transferred from the patch to the cover material with the lapse of time, and the drug transferred to the cover material is directly released to the skin. In this regard, the present inventors have also found the following fact. Specifically, for example, a patch which is desirably attached for a long period, such as a patch using the above-described rivastigmine as the drug, has to gradually and continuously release the drug to the skin to keep the blood concentration of the drug constant for a long period (preferably for 3 to 7 days or longer). Hence, such a patch is especially required to be excellent in sustained-release. However, the direct release of the drug from the cover material to the skin as described above impairs the sustained-release of the drug, so that the amount of the drug released and the amount of the drug permeating through the skin may increase temporarily and sharply immediately after the patch is attached, and then decrease rapidly. Hence, it is difficult to keep the blood concentration of the drug constant for a long period.

The present invention has been made in view of the problems of the above-described conventional techniques, and an object of the present invention is to provide a cover material-equipped patch and a cover material-equipped patch kit which can be attached for a long period, and with which the transfer of the drug from the patch to the cover material is sufficiently reduced.

[Solution to Problem]

The present inventors have conducted intensive study to achieve the above-described object, and consequently have found the following fact. Specifically, a cover material-equipped patch comprising: a patch comprising a support layer and a drug reservoir layer; and a cover material comprising a cover layer and an adhesive layer, wherein the drug reservoir layer contains a drug and a drug reservoir base agent, and the adhesive layer contains an adhesive base agent, and a relationship among a solubility parameter of the drug (hereinafter, abbreviated as "SPa" in some cases), a solubility parameter of the drug reservoir base agent (hereinafter, abbreviated as "SPb" in some cases), and a solubility parameter of the adhesive base agent (hereinafter, abbreviated as "SPc" in some cases) satisfies specific conditions can be attached for a long period owing to the cover material and also makes it possible to sufficiently reduce the transfer of the drug from the drug reservoir layer (patch) to the adhesive layer (Cover Material) during storage and/or application, although the cover material and the patch are stacked on each other.

In general, there is a tendency that the greater the difference in solubility parameter (SP value) between two components is, the less soluble in each other the two components are. In contrast, the present inventors have found that the transfer of the drug from the drug reservoir layer to the adhesive layer is sufficiently reduced in a cover material-equipped patch and a cover material-equipped patch kit, when the difference (SPa-SPc) between SPa and SPc is not smaller than a specific valve, the difference (SPa-SPb) between SPa and SPb is not larger than a specific valve, and, astonishingly, the (SPa-SPc) is not larger than a specific valve. More astonishingly, the present inventors have found that, to achieve such an effect, the range of the difference (SPb-SPc) between SPb and SPc has to be a specific range which is not identical to the range derived from the above-described relationships. These findings have led to the completion of the present invention.

Specifically, a cover material-equipped patch of the present invention is a cover material-equipped patch comprising:

a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$0 \leq |SPa-SPb| \leq 1.5 \cdots (1)$,
$2.0 \leq (SPa-SPc) \leq 2.8 \cdots (2)$, and
$1.0 \leq (SPb-SPc) \leq 2.8 \cdots (3)$.

The cover material-equipped patch of the present invention is preferably such that the formula (1) is the following formula:

$$0 \leq (SPa-SPb) \leq 1.5 \cdots (1').$$

In addition, in the cover material-equipped patch of the present invention, the drug is preferably at least one selected from the group consisting of rivastigmine, terbinafine, emedastine, and pharmaceutically acceptable salts thereof.

Further, it is preferable that the drug reservoir base agent contain an acrylic adhesive agent, and it is more preferable that the acrylic adhesive agent be a polymer obtained by polymerization of at least one selected from the group consisting of (meth)acrylic acid, 2-ethylhexyl (meth)acrylate, methyl (meth)acrylate, butyl (meth)acrylate, and hydroxyethyl (meth)acrylate.

In addition, it is preferable that the adhesive base agent contain a rubber-based adhesive agent, and it is more preferable that the rubber-based adhesive agent be at least one selected from the group consisting of styrene-isoprene-styrene block copolymer, styrene-butadiene-styrene block copolymer, styrene-ethylene-butylene-styrene block copolymer, natural rubber, polyisobutylene, and polyisoprene.

Moreover, the cover material-equipped patch of the present invention is preferably such that an amount of the drug transferred from the drug reservoir layer to the adhesive layer during storage at a temperature of 60°C for 2 weeks is 3% by mass or less of the drug contained in the drug reservoir layer before the storage.

A cover material-equipped patch kit of the present invention is a cover material-equipped patch kit comprising:

a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are to be arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa - SPb| \leq 1.5 \cdots (1),$$

$$2.0 \leq (SPa - SPc) \leq 2.8 \cdots (2),$$

and

$$1.0 \leq (SPb - SPc) \leq 2.8 \cdots (3).$$

Note that, in the present invention, the solubility parameter (SP value) of each component is a value $\delta$ [ (MPa)$^{1/2}$] obtainable by the method described in Fedors R. F., A method for estimating both the solubility parameters and molar volumes of liquids, Poly. Eng. Sci., 1974, 14, pp. 147-154 by using the following Fedors' calculation formula:

$$\delta = (\Sigma \Delta ei / \Sigma \Delta vi)^{1/2}.$$

The solubility parameter (SP value) can be determined as the square root of the ratio of the total sum ($\Delta ei$) of the energy of vaporization of atoms or groups of atoms in the chemical structure of the compound to the total sum ($\Delta vi$) of the molar volumes. In addition, in the present invention, the solubility parameter (SPa) of the drug is the SP value of the drug (or the SP value of a mixture of components, when the drug is the mixture of components). The solubility parameter (SPb) of the drug reservoir base agent is the SP value of an adhesive agent or an additive described below (or the SP value of a mixture of adhesive agent (s) and/or additive (s) described below, when the drug reservoir base agent is the mixture of components), among all the components contained in the drug reservoir layer. The solubility parameter (SPc) of the adhesive base agent is the SP value of an adhesive agent or an additive described below (or the SP value of the mixture of adhesive agent (s) and/or additive(s), when the adhesive base agent is the mixture of components), among all the components contained in the adhesive layer. In addition, in the present invention, (meth) acrylic acid refers to acrylic acid and methacrylic acid.

[Advantageous Effects of Invention]

According to the present invention, it is possible to provide a cover material-equipped patch and a cover material-equipped patch kit which can be attached for a long period, and with which the transfer of the drug from the patch to the cover material is sufficiently reduced.

[Brief Description of Drawings]

[Fig. 1] Fig. 1 is a schematic cross-sectional view showing a preferred embodiment of a cover material-equipped patch of the present invention.
[Fig. 2] Fig. 2 is a schematic cross-sectional view showing another preferred embodiment of the cover material-equipped patch of the present invention.

[Description of Embodiments]

Hereinafter, a detailed description will be made by showing preferred embodiments of the present invention as examples. First, a cover material-equipped patch of the present invention will be described in detail based on preferred embodiments thereof. The cover material-equipped patch of the present invention is a cover material-equipped patch comprising:

a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa - SPb| \leq 1.5 \cdots (1),$$

$$2.0 \leq (SPa - SPc) \leq 2.8 \cdots (2),$$

and

$$1.0 \leq (SPb - SPc) \leq 2.8 \cdots (3).$$

<Patch>

The patch according to the present invention comprises a support layer and a drug reservoir layer stacked on one surface of the support layer.

(Support Layer)

The support layer according to the present invention is not particularly limited, as long as the support layer can support the drug reservoir layer. Any known support layer for a patch can be employed, as appropriate, as the support layer according to the present invention. Examples of the material of the support layer include synthetic resins such as polyesters including polyethylene terephthalate, polybutylene terephthalate, and polyethylene naphthalate; polyolefins such as polyethylene and polypropylene; polyurethanes; and ethylene-vinyl acetate copolymer, as well as metals such as aluminum, and paper. In addition, the form of the support layer made of such a material is, for example, a film; a sheet such as a foamed sheet, a porous sheet, or a microporous sheet; a fabric such as a woven fabric, a knitted fabric, or a nonwoven fabric; foil; or a laminate of any ones of them. The support layer according to the present invention is preferably impermeable to the drug from the viewpoint of more sufficiently reducing the transfer of the drug from the drug reservoir layer to the adhesive layer. Especially, the support layer is preferably a polyester film from the viewpoint that a polyester film has excellent flexibility and excellent drug impermeability. In addition, a thickness of the support layer is not particularly limited, either, and is preferably about 2 to 600 $\mu$m, in general. The thickness of the support layer is more preferably 5 to 100 $\mu$m, from the viewpoint that when the cover material is stacked on the support layer, the support layer can come into sufficiently close contact with the cover material, while keeping a sufficient strength, so that the patch can be attached to the skin for a longer period.

(Drug reservoir layer)

The drug reservoir layer according to the present invention contains a drug and a drug reservoir base agent. The drug reservoir layer has a thickness which results in preferably 20 to 500 g/m$^2$, and more preferably 50 to 300 g/m$^2$. If the thickness of the drug reservoir layer is less than the lower limit, it tends to be difficult to maintain a sufficient skin permeation rate of the drug for a long time. Meanwhile, if the thickness exceeds the upper limit, the so-called cold flow of the drug reservoir layer is more likely to occur, and it tends to be difficult for the drug reservoir layer to keep the sufficient thickness and the shape.

[Drug]

The drug according to the present invention is not particularly limited in terms of the drug action, and examples of the drug include hypnotic and sedative drugs (flurazepam, rilmazafone, phenobarbital, amobarbital, medetomidine, dexmedetomidine, and the like), antipyretic and antiinflammatory agents (butorphanol, perisoxal, acetaminophen, mefenamic acid, diclofenac sodium, aspirin, alclofenac, ketoprofen, flurbiprofen, naproxen, piroxicam, pentazocine, indomethacin, felbinac, glycol salicylate, aminopyrine, loxoprofen, meloxicam, lornoxicam, and the like), steroidal anti-inflammatory agents (hydrocortisone, prednisolone, dexamethasone, betamethasone, and the like), analeptics and stimulants (methamphetamine, amphetamine, methylphenidate, and the like), neuropsychiatric agents (imipramine, diazepam, sertraline, fluvoxamine, paroxetine, citalopram, fluoxetine, alprazolam, haloperidol, clomipramine, amitriptyline, desipramine, amoxapine, maprotiline, mianserin, setiptiline, trazodone, lofepramine, milnacipran, duloxetine, venlafaxine, chlorpromazine, thioridazine, diazepam, meprobamate, etizolam, risperidone, asenapine, and the like), hormone drugs (estradiol, estriol, progesterone, norethisterone, metenolone, testosterone, and the like),local anesthetics (lidocaine, procaine, tetracaine, dibucaine, propitocaine, and the like), agents for urinary organs (oxybutynin, tamsulosin, propiverine, imidafenacin, solifenacin, tolterodine, and the like), skeletal muscle relaxants (tizanidine, eperisone, pridinol, suxamethonium, and the like), agents for reproductive organs (ritodrine and meluadrine), antiepileptic agents (sodium valproate, clonazepam, carbamazepine, and the like), autonomic agents (carpronium, neostigmine, bethanechol, and the like), antiparkinsonian agents (pergolide, bromocriptine, trihexyphenidyl, amantadine, talipexole, cabergoline, droxidopa, biperiden, selegiline, and the like), diuretic agents (hydroflumethiazide, furosemide, and the like), respiratory stimulants (lobeline, dimorpholamine, naloxone, and the like), antimigraine agents (dihydroergotamine, sumatriptan, ergotamine, flunarizine, cyproheptadine, and the like), antihistamines (clemastine, diphenhydramine, chlorpheniramine, diphenylpyraline, promethazine, and the like), bronchodilators (tulobuterol, procaterol, salbutamol, clenbuterol, fenoterol, terbutaline, isoprenaline, formoterol, and the like), cardiotonics (isoprenaline, dopamine, and the like), coronary vasodilators (diltiazem, verapamil, isosorbide, nitroglycerin, nicorandil, and the like), peripheral vasodilators (nicametate, tolazoline, and the like), smoking cessation aids (nicotine, varenicline, and the like), agents for circulatory organs (flunarizine, nicardipine, nitrendipine, nisoldipine, felodipine, amlodipine, nifedipine, nilvadipine, manidipine, benidipine, enalapril, temocapril, alacepril, imidapril, cilazapril, lisinopril, captopril, trandolapril, perindopril erbumine, atenolol, bisoprolol, metoprolol, betaxolol, arotinolol, celiprolol, carvedilol, carteolol, bevantolol, valsartan, candesartan cilexetil, losartan potassium, clonidine, and the like), antiarrhythmicagents (propranolol, alprenolol, procainamide, mexiletine, nadolol, disopyramide, and the like), anti-malignant-ulcer agents (cyclophosphamide, fluorouracil, tegafur, procarbazine, ranimustine, irinotecan, fluridine, and the like), antilipemic agents (pravastatin, simvastatin, bezafibrate, probucol, and the like), hypoglycemic agents (glibenclamide, chlorpropamide, tolbutamide, glymidinesodium, glybuzole, buformin, and the like), anti-peptic ulcer agents (proglumide, cetraxate, spizofurone, cimetidine, glycopyrronium, and the like), cholagogues (ursodesoxycholic acid, osalmid, and the like), gastroprokinetic agents (domperidone, cisapride, and the like), agents for hepatic diseases (tiopronin and the like), anti-allergic agents (ketotifen, azelastine, emedastine, and the like), antiviral agents (acyclovir and the like), antivertigoagents (betahistine, difenidol, and the like), antibiotics (cephaloridine, cefdinir, cefpodoxime proxetil, cefaclor, clarithromycin, erythromycin, methylerythromycin, kanamycin, cycloserine, tetracycline, benzylpenicillin potassium, propicillin potassium, cloxacillin sodium, ampicillin sodium, bacampicillin, carbenicillin sodium, chloramphenicol, and the like), agents for habitual intoxication (cyanamide and the like), appetite suppressants (mazindol and the like), chemotherapeutic agents (isoniazid, ethionamide, pyrazinamide, and the like), blood coagulation accelerators (ticlopidine, warfarin potassium, and the like), anti Alzheimer's agents (physostigmine, donepezil, tacrine, arecoline, xanomeline, galantamine, rivastigmine, and the like), serotonin receptor antagonist antiemetics (ondansetron, granisetron, ramosetron, azasetron, and the like), antigout agents (colchicine, probenecid, sulfinpyrazone, and the like), narcotic analgesics (morphine, morphine, codeine, cocaine, pethidine, and the like), and antifungal agents (terbinafine, butenafine, amorolfine, neticonazole, miconazole, luliconazole, itraconazole, liranaftate, and the like). One of these drugs may be used alone, or two or more thereof may be used in combination.

In addition, the drug according to the present invention may be in a free form (free base) or may be a pharmaceutically acceptable salt of the drug. The drug in the free form may be one blended in the free form at the production, or may be one formed by desalination of a pharmaceutically acceptable salt of the drug with a desalting agent during the production and/or in a produced pharmaceutical preparation. The drug may be in one of these forms or a mixture of two or more thereof. Examples of the pharmaceutically acceptable salts of the drug include salts with metals such as alkali metals, alkaline earth metals, and aluminum; salts with amines such as tromethamine; salts with protic acids such as hydrochloric acid, sulfuric acid, acetic acid, nitric acid, hydrobromic acid, phosphoric acid, methanesulfonicacid, fumaricacid, maleic acid, citric acid, tartaric acid, besylic acid, succinic acid, and tannic acid; and chlorides. Note that examples of the desalting agent include alkali metal hydroxides such as sodium hydroxide, potassium hydroxide, and magnesium hydroxide.

In addition, the drug according to the present invention is preferably at least one selected from the group consisting of rivastigmine, terbinafine, emedastine, and pharmaceutically acceptable salts thereof. Of these drugs, at least one selected from the group consisting of rivastigmine and pharmaceutically acceptable salts thereof, which may be continuously administered for relatively long periods, is more preferable from the viewpoint that the present invention is effective especially when a drug which has to be continuously administered for a long period is used.

In addition, especially when rivastigmine is used as the drug, the drug is highly likely to be transferred from the drug reservoir layer to the adhesive layer. Against this problem, the present inventors have found that the present invention makes it possible to sufficiently reduce the transfer of a drug, even when the drug is highly likely to be transferred from the drug reservoir layer to the adhesive layer, as described above. Accordingly, also from the viewpoint that a further remarkable effect can be achieved by the configuration of the present invention, the drug is preferably at least one selected from the group consisting of rivastigmine and pharmaceutically acceptable salts thereof. In addition, the form of the drug according to the present invention is more preferably in a free form from the viewpoint of better skin permeability. In the present invention, even when a drug in a free form having an excellent releasability from a patch is used as described above, the transfer to the adhesive layer is sufficiently reduced, and hence the release from the cover material to the skin is sufficiently reduced.

A general content of the drug in the patch according to the present invention cannot be specified, because the content varies depending on the type of the drug and the purpose of the therapy. However, the content of the drug is preferably an amount which is equivalent to 2 to 50% by mass in terms of free form in the drug reservoir layer yet to be stacked on the cover material. From the viewpoints that the amount of the drug transferred from the drug reservoir layer to the adhesive layer tends to further decrease, and that a sufficient skin permeation rate of the drug tends to be maintained for a longer time, the content of the drug is more preferably an amount which is equivalent to 5 to 40% by mass in terms of free form. For example, when rivastigmine and/or a salt thereof is used as the drug according to the present invention, a total content thereof is more preferably an amount which is equivalent to 10 to 40% by mass in terms of free form in the drug reservoir layer yet to be stacked on the cover material, from the viewpoint that a sufficient skin permeation rate of the drug tends to be maintained for a longer time.

[Drug Reservoir Base Agent]

The drug reservoir base agent according to the present invention is a base agent capable of retaining the form of the drug reservoir layer. In the present invention, the drug reservoir base agent refers to a mixture or pure substance which consists of all the components, except the drug, contained in the drug reservoir layer yet to be stacked on the cover material. The drug reservoir base agent contains an adhesive agent (s) and/or an additive(s). From the viewpoints of enabling the patch to be attached for a long period and also of improving the adhesion to the skin, the drug reservoir base agent preferably contains an adhesive agent capable of providing the drug reservoir layer itself with adhesiveness to the skin. Note that, in the present invention, the adhesive agent refers to a compound capable of expressing adhesiveness at a temperature (preferably 0°C to 50°C, more preferably 10°C to 40°C, and further preferably 15°C to 40°C) at which the patch is applied. Examples of the adhesive agent include rubber-based adhesive agents, acrylic adhesive agents, and silicone-based adhesive agents. One of these adhesive agents may be used alone, or two or more thereof may be used in combination.

Examples of the rubber-based adhesive agents include styrene-based block copolymers such as styrene-isoprene-styrene block copolymer (hereinafter, abbreviated as "SIS" in some cases), styrene-butadiene-styrene block copolymer (hereinafter, abbreviated as "SBS" in some cases, and styrene-ethylene-butylene-styrene block copolymer; natural rubber; polyisobutylene (hereinafter, abbreviated as "PIB" in some cases); and polyisoprene. One of these rubber-based adhesive agents may be used alone, or two or more thereof may be used in combination.

Examples of the acrylic adhesive agents include acrylic polymers each obtained by polymerization of at least one of or copolymerization of at least two or more of (meth) acrylic monomers such as (meth) acrylic acid, 2-ethylhexyl(meth)acrylate,methyl(meth)acrylate,butyl (meth)acrylate, and hydroxyethyl (meth)acrylate. Examples of the acrylic polymers include 2-ethylhexyl acrylate-vinyl acetate copolymer, 2-ethylhexyl acrylate-vinyl acetate-acrylic acid copolymer, 2-ethylhexyl acrylate-vinyl acetate-hydroxyethyl acrylate copolymer, 2-ethylhexyl acrylate-vinyl acetate-hydroxyethyl acrylate-acrylic acid copolymer, 2-ethylhexyl acrylate-2-ethylhexyl methacrylate-dodecyl methacrylate copolymer, and the like. One of these acrylic polymers may be used alone, or two or more thereof may be used in combination. In addition, commercially available products such as those of the DURO-TAK acrylic adhesive agent series (manufactured by Henkel AG & Co. KGaA) may also be used as these acrylic adhesive agents.

As the silicone-based adhesive agent, it is preferable to use a polymer having an organopolysiloxane backbone. In addition, when the polymer having an organopolysiloxane backbone has hydroxy groups (for example, silanol groups), it is more preferable that at least some of the hydroxy groups be capped with trimethylsilyl groups. In addition, the polymer having an organopolysiloxane backbone further preferably has adhesiveness. Note that the capping with the trimethylsilyl groups includes a mode of end-capping of terminal silanol groups of the polymer having an organopolysiloxane backbone with trimethylsilyl groups. Examples of the polymer having an organopolysiloxane backbone include polydimethylsiloxane (a polymer designated by MQ according to the designation of ASTMD-1418 or the like), polymethylvinylsiloxane (a polymer designated by VMQ according to the designation of ASTMD-1418 or the like), polymethylphenylsiloxane (a polymer designated by PVMQ according to the designation of ASTMD-1418 or the like), and the like. One of these polymers may be used alone, or two or more thereof may be used in combination. In addition, commercially available products such as those of the PSA adhesive agent series (manufactured by Dow Corning Corporation) may also be used as these silicone-based adhesive agents.

In the patch according to the present invention, it is preferable to use at least one of the above-described acrylic adhesive agents as the adhesive agent, from the viewpoint that there is a tendency that it is possible to blend the drug in the adhesive agent in such an amount that a sufficient skin permeation rate can be maintained for a longer time.

In addition, in the patch according to the present invention, a general content of the adhesive agent is not presented, because the content of the adhesive is adjusted so that the solubility parameter of the drug reservoir base agent can be within a range where the conditions described later are satisfied. From the viewpoint that an excellent adhesion to the skin tends to be achieved, the content of the adhesive is preferably an amount which is equivalent to 50 to 98% by mass in the drug reservoir layer yet to be stacked on the cover material.

The drug reservoir base agent according to the present invention may further contain the above-described additives, in addition to the adhesive agent. In the present invention, the additives refer to tackifiers, softeners, and solubilizers. One of these additives may be used alone, or two or more thereof may be used in combination. For example, when styrene-based block copolymer and/or natural rubber is used as the adhesive agent, the drug reservoir base agent preferably further contains at least one selected from the group consisting of tackifiers and softeners.

Examples of the tackifier include rosin resins, rosin ester resins, terpene resins, terpene phenolic resins, C5-type petroleum resins, C5/C9-type petroleum resins, DCPD (dicyclopentadiene)-type petroleum resins, coumarone-indene resins, alicyclic saturated hydrocarbon resins (hereinafter, abbreviated as "AP" in some cases), and hydrogenated product thereof. One of these tackifiers may be used alone, or two or more thereof may be used in combination. A general content of the tackifier cannot be specified, because the content of the tackifier is adjusted so that the solubility parameter of the drug reservoir base agent can be within a range where the conditions described later are satisfied. However, the content of the tackifier is preferably an amount which is equivalent to 0 to 48% by mass in the drug reservoir layer yet to be stacked on the cover material.

The softeners include petroleum-based oils (examples : paraffinic process oils such as liquid paraffin (hereinafter, abbreviated as "LP" in some cases), naphthenic process oils, aromatic process oils, and the like), squalane, squalene, vegetable-based oils (examples: olive oil, camellia oil, castor oil, tall oil, peanut oil, and the like), silicone oils, diprotic acid esters (examples: dibutyl phthalate, dioctyl phthalate, and the like), liquid rubbers (examples: liquidpolybutene, liquid isoprene rubber, and the like), liquid fatty acid esters (examples: isopropyl myristate, hexyl laurate, diethyl sebacate, diisopropyl sebacate, and the like), diethylene glycol, polyethylene glycol, glycol salicylate, propylene glycol, dipropylene glycol, triacetin, triethyl citrate, crotamiton, and the like. One of these softeners may be used alone, or two or more thereof may be used in combination. Of these softeners, liquid paraffin, liquid polybutene, isopropyl myristate, diethyl sebacate, and hexyl laurate are preferable, and liquid paraffin is more preferable. A general content of the softener cannot be specified, because the content of the softener is adjusted so that the solubility parameter of the drug reservoir base agent can be within a range where the conditions described later are satisfied. However, the content of the softener is preferably an amount which is equivalent to 0 to 48% by mass in the drug reservoir layer yet to be stacked on the cover material.

Although it depends on the type of the solute to be dissolved, examples of the solubilizers include fatty acids (examples: capric acid, oleic acid, linoleic acid, and the like), fatty acid esters (examples: isopropyl myristate, isopropylpalmitate, and the like), fatty acid derivatives (examples: propylene glycol monolaurate, lauric acid diethanolamide, and the like), glycerin fatty acid esters (examples: glycerin monolaurate, glycerin monooleate, and the like), polyol fatty acid esters (examples: sorbitan monolaurate and the like), aliphatic alcohols (examples: octyldodecanol, isostearylalcohol, oleylalcohol, and the like), polyols (examples: propylene glycol, dipropylene glycol, polyethylene glycol, and the like), pyrrolidone derivatives (examples: N-methyl-2-pyrrolidone and the like), organic acids (examples: acetic acid, lactic acid, and the like), and organic acid salts (examples: sodium acetate, sodium lactate, and the like). One of these solubilizers may be used alone, or two or more thereof may be used in combination. A general content of the solubilizer cannot be specified, because the content of the solubilizer is adjusted so that the solubility parameter of the drug reservoir base agent can be within a range where the conditions described later are satisfied. However, the content of the solubilizer is preferably an amount which is equivalent to 0 to 20% by mass and more preferably an amount which is equivalent to 0.1 to 20% by mass in the drug reservoir layer yet to be stacked on the cover material.

In addition, the total content of the adhesive agent and the additive in the patch according to the present invention is preferably an amount which is equivalent to 50 to 98% by mass in the drug reservoir layer yet to be stacked on the cover material.

The drug reservoir base agent according to the present invention may further contain a bulking agent, a stabilizer, and the like, in addition to the adhesive agent and/or the additives. The drug reservoir base agent according to the present invention may further contain the desalting agent or a metal salt produced from the pharmaceutically acceptable salts of the drug and the desalting agent.

Examples of the bulking agent include inorganic compounds such as silica, aluminum oxide, aluminum hydroxide, zinc oxide, titanium oxide, talc, clay, kaolin, glass, barium sulfate, calcium carbonate, hydroxyapatite, and ceramics; and organic compounds such as cellulose, silk, polyesters, polyolefins, polyacrylates, polymethacrylates, and polystyrene. One of these bulking agents may be used alone, or two or more thereof may be used in combination.

Meanwhile, examples of the stabilizer include tocopherols, ester derivatives of tocopherols, ascorbic acid, ascorbic acid stearate, nordihydroguaiaretic acid, dibutylhydroxytoluene (hereinafter, abbreviated as "BHT" in some cases), butylhydroxyanisole, and the like. One of these stabilizers may be used alone, or two or more thereof may be used in combination. When the bulking agent and/or the stabilizer are contained in the drug reservoir base agent, a general content thereof cannot be specified, because the content is adjusted so that the solubility parameter of the drug reservoir base agent can be within a range where the conditions described later are satisfied. However, the content of each of the bulking agent and the stabilizer is preferably an amount which is equivalent to 5% by mass or less in the drug reservoir layer yet to be stacked on the cover material.

Examples of the metal salt include sodium chloride, sodium citrate, sodium fumarate, and the like.

(Release liner)

The patch according to the present invention more preferably has a structure in which a release liner is further stacked on a surface of the drug reservoir layer on the side opposite from the support layer, from the viewpoint of protecting the drug reservoir layer until its use. The release liner is not particularly limited, and a known release liner for a patch can be employed, as appropriate. The release liner may be a film of a polyester such as polyethylene terephthalate or a film of polyvinyl chloride, polyvinylidene chloride, or the like; a laminate film of woodfree paper and a polyolefin film; paper; or a laminate of any ones of them. The release liner is preferably subjected to a release treatment such as silicone coating to facilitate the detachment of the release liner. In addition, a thickness of the release liner is not particularly limited, and is preferably about 2 to 300 $\mu$m, in general.

(Patch Adhesive Layer)

It is also preferable that the patch according to the present invention have a structure (multilayer type patch) in which an adhesive layer (hereinafter, referred to as a patch adhesive layer to be distinguishable from the adhesive layer in the cover material) is further stacked on a surface of the drug reservoir layer on the side opposite from the support layer (between the drug reservoir layer and the release liner, when the release liner is further provided), from the viewpoint that there is a tendency that the content of the drug in the drug reservoir layer can be further increased, while the attachment for a longer period can be achieved, and also the adhesion of the patch to the skin is improved. The patch adhesive layer is not particularly limited, and a known adhesive layer for a multilayer type patch can be employed, as appropriate, within a range not impairing an effect of the present invention. For example, the patch adhesive layer may be a layer containing at least one adhesive agent selected from the group consisting of rubber-based adhesive agents, acrylic adhesive agents, and silicone-based adhesive agents. If necessary, the patch adhesive layer may further contain additives, bulking agents, stabilizers, and the like. Examples of the rubber-based adhesive agents, the acrylic adhesive agents, the silicone-based adhesive agents, the additives, the bulking agents, and the stabilizers are the same as those listed for the drug reservoir base agent of the patch. In addition, a thickness of the patch adhesive layer is not particularly limited, and the patch adhesive layer preferably has a thickness which results in 5 to 200 g/m$^2$, from the viewpoints of maintaining a sufficient skin permeation rate of the drug and of further preventing the cold flow from occurring.

<Cover Material>

The cover material according to the present invention comprises a cover layer; and an adhesive layer (hereinafter, referred to as a cover material adhesive layer in some cases) which is stacked on one surface of the cover layer. The cover material according to the present invention is capable of fixing the patch to the skin, and is preferably one which fixes the patch to the skin while covering the patch entirely. A size of the cover material is preferably adjusted to a size with which the adhesive layer of the cover material can cover a peripheral portion or end portions of the patch. In addition, the shape of the cover material is not particularly limited, and, for example, may be a quadrilateral, circular, or elliptical shape.

(Cover Layer)

The cover layer according to the present invention is not particularly limited, as long as the cover layer can support the adhesive layer and can protect the adhesive layer and the patch during application. Examples of the cover layer include those listed for the support layer of the patch. Of these cover layers, a polyester film is preferable, from the viewpoint that the amount of the drug transferred from the drug reservoir layer to the adhesive layer is further reduced. In addition, a thickness of the cover layer is not particularly limited, and is preferably about 2 to 300 $\mu$m, in general.

(Adhesive Layer)

The adhesive layer (cover material adhesive layer) according to the present invention is a layer containing an adhesive base agent. The adhesive layer preferably has a thickness which results in 5 to 300 $g/m^2$, and more preferably a thickness which results in 10 to 200 $g/m^2$. If the thickness of the adhesive layer is less than the lower limit, the adhesiveness tends to decrease. Meanwhile, if the thickness exceeds the upper limit, the adhesive layer becomes more susceptible to the so-called cold flow, and it tends to be difficult to keep a sufficient thickness and the shape.

[Adhesive Base Agent]

The adhesive base agent according to the present invention is a base agent capable of maintaining the form of the adhesive layer, and refers to a mixture or pure substance which consists of all the components contained in the adhesive layer yet to be stacked on the patch. In addition, the adhesive base agent according to the present invention contains an adhesive agent for fixing the cover material and the patch to the skin. Examples of the adhesive agent contained in the adhesive base agent according to the present invention include the adhesive agents listed for the drug reservoir base agent of the patch. As the adhesive agent in the cover material according to the present invention, it is preferable to use at least one of the above-described rubber-based adhesive agents, from the viewpoint that the amount of the drug transferred from the drug reservoir layer to the adhesive layer tends to be further reduced.

A general content of the adhesive agent in the cover material according to the present invention cannot be specified, because the content of the adhesive agent is adjusted so that the solubility parameter of the adhesive base agent can be within a range where the conditions described later are satisfied. The content of the adhesive agent is preferably an amount which is equivalent to 75 to 100% by mass in the adhesive layer yet to be stacked on the patch, from the viewpoint that there is a tendency that the adhesive agent remaining on the skin after the cover material attached to the skin is detached can be eliminated or reduced, and excellent adhesion to the skin can be achieved.

In addition, the adhesive base agent according to the present invention may further contain additives within a range not impairing an effect of the present invention. Examples of the additives include the additives listed for the drug reservoir base agent of the patch. Moreover, the adhesive base agent according to the present invention may further contain bulking agents, stabilizers, and the like within a range not impairing an effect of the present invention. Examples of the bulking agents and the stabilizers include those listed for the drug reservoir base agent of the patch. In addition, a general preferred content of each of these additives, bulking agents, and stabilizers in the adhesive layer (a content in the adhesive layer yet to be stacked on the patch) cannot be specified, because the content is adjusted so that the solubility parameter of the adhesive base agent can be within a range where the conditions described later are satisfied. However, the content of the tackifier is preferably 0 to 25% by mass, the content of the softener is preferably 0 to 25% by mass, the content of the solubilizer is preferably 0 to 20% by mass (more preferably 0.1 to 20% by mass), and the contents of the bulking agent or the stabilizer are each preferably 0 to 5% by mass.

(Release Liner)

The cover material according to the present invention more preferably has a structure in which a release liner is further stacked on a surface of the adhesive layer on the side opposite from the cover layer, from the viewpoint of protecting the adhesive layer until the adhesive layer is stacked on the patch and/or until the cover material-equipped patch is used. The release liner is not particularly limited, and any of the release liners listed as the release liner of the patch can be used.

<Cover Material-Equipped Patch>

Hereinafter, a configuration of the cover material-equipped patch of the present invention will be described in detail with reference to the drawings by showing preferred embodiments of the present invention as examples; however, the present invention is not limited thereto. Note that, in the following description and drawings, the same or equivalent components are denoted by the same reference numerals , and overlapping description thereof is omitted.

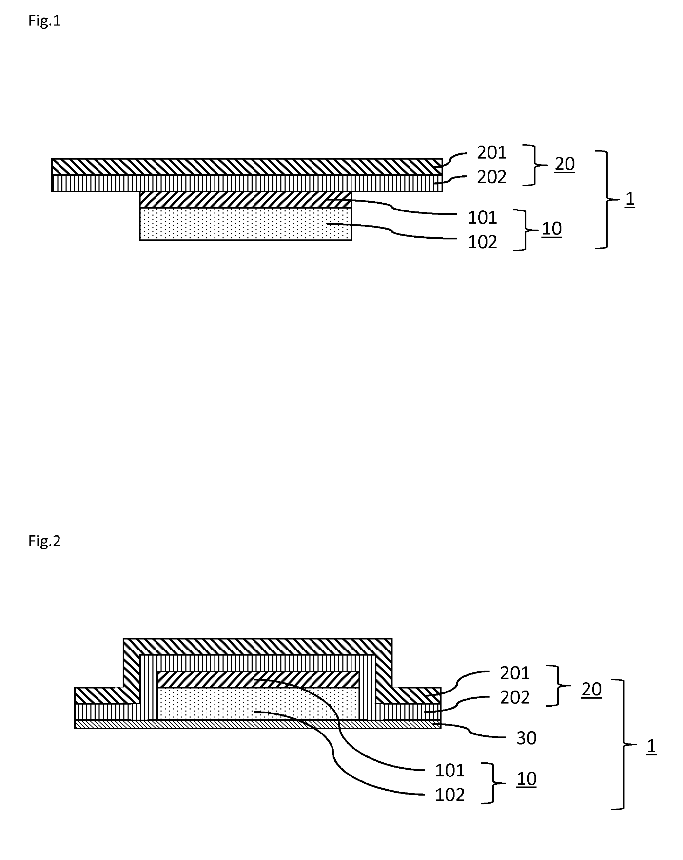

In a cover material-equipped patch 1 of the present invention, a patch 10 and a cover material 20 are arranged in such a manner that an adhesive layer 202 is stacked on a surface of a support layer 101 on a side opposite from a drug reservoir layer 102, as shown in Fig. 1. In addition, for example, the cover material-equipped patch 1 of the present invention may have such an arrangement that exposed side surfaces of the drug reservoir layer 102 and a surface of the adhesive layer 202 on a side opposite from a cover layer 201 can be in direct contact with each other as shown in Fig. 2, from the viewpoint that the adhesion of the cover material-equipped patch to the skin is further improved by bringing the patch and the cover material into close contact with each other. The cover material-equipped patch 1 of the present invention may further comprise a release liner 30. Even with the arrangement in which the drug reservoir layer 102 and the adhesive layer 202 are in direct contact with each other, the present invention makes it possible to sufficiently reduce the transfer of the drug from the drug reservoir layer 102 to the adhesive layer 202. Note that when the arrangement in which the drug reservoir layer 102 and the adhesive layer 202 are in direct contact with each other as described above is employed, a contact area between the drug reservoir layer 102 and the adhesive layer 202 is preferably 10% or less and more preferably 5% or less of the area of the surface of the adhesive layer 202 on the side opposite from the cover layer 201, from the viewpoint that the transfer of the drug can be reduced more sufficiently.

In the cover material-equipped patch 1 of the present invention, sizes of the patch 10 and the cover material 20 are not particularly limited, as long as the cover material 20 can fix the patch 10 to the skin. From the viewpoint that the transfer of the drug from the drug reservoir layer 102 to the adhesive layer 202 can be reduced more sufficiently, the sizes are such that a mass ratio of the drug reservoir layer 102 in the patch 10 and the adhesive layer 202 in the cover material 20 (the mass of the drug reservoir layer 102:the mass of the adhesive layer 202) can be preferably 100:1 to 1:10, and more preferably 30:1 to 1:3.

In the cover material-equipped patch of the present invention, a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa-SPb| \leq 1.5 \cdots (1),$$

$$2.0 \leq (SPa-SPc) \leq 2.8 \cdots (2),$$

and

$$1.0 \leq (SPb-SPc) \leq 2.8 \cdots (3).$$

In the formula (1), "|SPa-SPb|" represents the absolute value of the difference (SPa-SPb) obtained by subtracting the solubility parameter (SPb) of the drug reservoir base agent from the solubility parameter (SPa) of the drug. As shown in the formula (1), the difference (SPa-SPb) obtained by subtracting SPb from SPa has to be within a range from -1.5 to 1.5. If the difference (SPa-SPb) between SPa and SPb is less than the lower limit, the resultant pharmaceutical preparation is affected more by sweat during application, because the hydrophilicity of the drug reservoir base agent is high. Meanwhile, if the difference (SPa-SPb) exceeds the upper limit, the amount of the drug transferred from the drug reservoir layer to the adhesive layer increases. The formula (1) is preferably the following formula (1'):

$$0 \leq (SPa-SPb) \leq 1.5 \cdots (1').$$

In other words, the difference (SPa-SPb) obtained by subtracting the solubility parameter (SPb) of the drug reservoir base agent from the solubility parameter (SPa) of the drug is preferably in a range from 0 to 1.5.

In the formula (2), "(SPa-SPc)" represents a difference obtained by subtracting the solubility parameter (SPc) of the adhesive base agent from the solubility parameter (SPa) of the drug. As shown in the formula (2), the difference (SPa-SPc) obtained by subtracting SPc from SPa has to be within a range from 2.0 to 2.8. If the difference (SPa-SPc) between SPa and SPc is less than the lower limit, the amount of the drug transferred from the drug reservoir layer to the adhesive layer increases. Meanwhile, the greater the difference in solubility parameter between two components is, the lower the solubility is. For this reason, presumably, the greater the difference between SPa and SPc is, the more the transfer of the drug from the drug reservoir layer to the adhesive layer is reduced, in general. However, the present inventors have found that when the difference between SPa and SPc exceeds the upper limit, the amount of the drug transferred from the drug reservoir layer to the adhesive layer increases. The difference (SPa-SPc) between SPa and SPc is particularly preferably within a range from 2.3 to 2.7, from the viewpoint that the transfer of the drug from the drug reservoir layer to the adhesive layer tends to be more sufficiently reduced.

In the formula (3), "(SPb-SPc)" represents a difference obtained by subtracting the solubility parameter (SPc) of the adhesive base agent from the solubility parameter (SPb) of the drug reservoir base agent. As shown in the formula (3), the difference (SPb-SPc) obtained by subtracting SPc from SPb has to be within a range from 1.0 to 2.8. If the difference (SPb-SPc) between SPb and SPc is either less than the lower limit or more than the upper limit, the amount of the drug transferred from the drug reservoir layer to the adhesive layer increases. Note that, for example, when the difference (SPa-SPb) between SPa and SPb is within the range from 0 to 1.5, the lower limit value of the difference between SPb and SPc is calculated to be 0.5 from the formulae (1') and (2). Meanwhile, the present inventors have found that if the difference between SPb and SPc is less than 1.0, the amount of the drug transferred from the drug reservoir layer to the adhesive layer increases. The difference (SPb-SPc) between SPb and SPc is particularly preferably within a range from 1.2 to 2.1 from the viewpoint that the transfer of the drug tends to be reduced more sufficiently.

The solubility parameters of the drug, the drug reservoir base agent, and the adhesive base agent are values obtained by using the Fedors' calculation formula, as described above. The solubility parameters can be adjusted by the composition of the drug or by the composition or the compositional ratio of the components contained in each base agent. General compositions cannot be specified, because the compositions depend on the type of the drug and the components contained in each base agent. However, for example, when at least one drug selected from the group consisting of rivastigmine, terbinafine, and emedastine is used as the drug, the drug reservoir base agent preferably contains an acrylic adhesive agent, and the adhesive base agent preferably contains a rubber-based adhesive agent.

When the solubility parameters of the drug, the drug reservoir base agent, and the adhesive base agent simultaneously satisfy the conditions represented by the formulae (1) to (3) as described above, the transfer of the drug from the patch to the cover material, i.e., from the drug reservoir layer to the adhesive layer is sufficiently reduced in the cover material-equipped patch of the present invention. In the cover material-equipped patch of the present invention as described above, for example, an amount of the drug transferred from the drug reservoir layer to the adhesive layer during storage at a temperature of 60°C for 2 weeks is preferably 3% by mass or less and more preferably 2.85% by mass or less, in terms of free form, of the drug contained in the drug reservoir layer before the storage. When the amount of the drug transferred is within the above-described range, it is possible to sufficiently suppress the sharp increase and decrease of the amount of the drug released and the amount of the drug permeating through the skin due to the direct release to the skin of the drug transferred to the cover material during storage and/or application, so that the blood concentration of the drug can be kept constant for a long period (preferably 3 to 7 days or longer).

<Methods for Producing Patch, Cover Material, and Cover Material-Equipped Patch>

The patch and the cover material according to the present invention can be obtained as follows. Specifically, a drug reservoir layer composition containing the drug, the drug reservoir base agent, and, if necessary, a solvent, and an adhesive layer composition containing the adhesive base agent and, if necessary, a solvent are prepared separately, so that the solubility parameter (SPa) of the drug, the solubility parameter (SPb) of the drug reservoir base agent, and the solubility parameter (SPc) of the adhesive base agent in a drug reservoir layer and an adhesive layer to be obtained can simultaneously satisfy the above-described conditions represented by the formulae (1) to (3). Then, the drug reservoir layer and the adhesive layer are formed by using these compositions. Methods for forming the drug reservoir layer and the adhesive layer as described above are not particularly limited, and the drug reservoir layer and the adhesive layer can be produced by known conventional methods. An example of the methods is as follows. Specifically, first, the drug reservoir layer composition (or the adhesive layer composition in the case of the cover material) is applied in a desired thickness onto one surface of the support layer (or the cover layer in the case of the cover material). Then, the solvent is removed to form the drug reservoir layer on the one surface of the support layer (or the adhesive layer on the one surface of the cover layer in the case of the cover material). Alternatively, when the patch and the cover material further comprise a release liner layer, it is also possible to first form the drug reservoir layer or the adhesive layer by applying a corresponding one of the compositions on one surface of the release liner layer and then stack the support layer or the cover layer on a surface of the drug reservoir layer or the adhesive layer on a side opposite from the release liner layer.

The solvent is not particularly limited, and can be selected, as appropriate, according to the types of the drug and the base agents. Examples of the solvents include lower alcohols such as methanol, ethanol, and isopropanol; toluene, xylene, pentane, n-hexane, cyclohexane, heptane, octane, methyl acetate, ethyl acetate, propyl acetate, methyl butyrate, ethyl butyrate, and propyl butyrate.

The cover material-equipped patch of the present invention can be produced by a known conventional method without any particular limitation. The cover material-equipped patch of the present invention may be produced by laminating the patch and the cover material, which are separately produced, to each other as shown in Fig. 1, or by forming the adhesive layer on a surface of the support layer on a side opposite from the drug reservoir layer, and then stacking the cover layer on the adhesive layer.

Meanwhile, a cover material-equipped patch kit of the present invention comprises the patch and the cover material. The patch and the cover material separately produced as described above are used as a cover material-equipped patch after the adhesive layer is arranged so as to be stacked on a surface of the support layer on a side opposite from the drug reservoir layer. This kit also makes it possible to attach the patch for a long period, and sufficiently reduce the transfer of the drug from the drug reservoir layer to the adhesive layer during application. In addition, the kit is preferable from the viewpoint that the drug is not transferred from the drug reservoir layer to the adhesive layer during storage.

[Examples]

Hereinafter, the present invention will be described more specifically on the basis of Examples and Comparative Examples; however, the present invention is not limited to Examples below. Note that the amount of the drug transferred was measured by the following method in each of Examples and Comparative Examples.

(Measurement of Amount of Drug Transferred)

The amount of a drug transferred of a cover material-equipped patch obtained in each of Examples and Comparative Examples was determined by the following method. Specifically, first, each cover material-equipped patch (patch: 5 cm$^2$, cover material: 15 cm$^2$) was sealed in an aluminum laminate pouch, and stored under an environment of a temperature of 60°C and a humidity of 75% for 2 weeks. Subsequently, the release liner was detached from each of the cover material-equipped patches stored for 2 weeks, and the patch and the cover material were separated from each other. The cover material separated from the patch was placed in a glass centrifuge tube, and 10 mL of tetrahydrofuran was added. Then, the adhesive layer was dissolved by shaking the tube. Subsequently, the adhesive base agent was precipitated by further adding 40 mL of methanol to the solution in which the adhesive layer was dissolved, and removed by filtration using a syringe filter. The amount (in terms of free form) of the drug contained in the solution after the filtration was determined by using a high-performance liquid chromatograph to determine the content of the drug in the adhesive layer of the cover material having been stored for 2 weeks. In addition, the content of the drug in the drug reservoir layer having been stored for 2 weeks was also determined in the same manner. Then, each amount of drug (drug transfer concentration) transferred from the drug reservoir layer to the adhesive layer was determined by using the following formula:

```
Drug transfer concentration [%]=(content of drug in
adhesive layer/(content of drug in adhesive layer+content
of drug in drug reservoir layer))×100.
```

(Patch Production Example 1)

First, a drug reservoir layer composition was obtained by adding 30 parts by mass of rivastigmine (free form) to a solution of an acrylic polymer 1 (an acrylic polymer having hydroxy (OH) groups, a drug reservoir base agent) in ethyl acetate (Duro-Tak 387-2516, manufactured by Henkel AG & Co. KGaA, acrylic polymer 1: 70 parts by mass). The obtained drug reservoir layer composition was applied onto one surface of a polyethylene terephthalate film (release liner) having been subjected to a release treatment. Then, ethyl acetate was removed by drying. Thus, a drug reservoir layer having a thickness of 100 g/m$^2$ was obtained. Subsequently, a patch 1 was obtained by stacking a polyethyleneterephthalate film (support layer) on a surface of the drug reservoir layer on the side opposite from the release liner.

(Patch Production Example 2)

A patch 2 was obtained in the same manner as in Patch Production Example 1, except that the composition of the drug reservoir layer was as follows:

[Drug Reservoir Base Agent]

acrylic polymer 2: 70 parts by mass, and [Drug]
rivastigmine (free form): 30 parts by mass.

Note that, as the acrylic polymer 2, a solution of an acrylic polymer having no functional group (acrylic polymer 2) in ethyl acetate (Duro-Tak 87-2194, manufactured by Henkel AG & Co. KGaA) was used with the acrylic polymer 2 being contained at 70 parts by mass.

(Patch Production Example 3)

First, a drug reservoir layer composition was obtained by stirring 70 parts by mass of polyisobutylene (PIB) (drug reservoir base agent) and 30 parts by mass of rivastigmine (free form) in toluene. The obtained drug reservoir layer composition was applied onto one surface of a polyethylene terephthalate film (release liner) having been subjected to a release treatment, and then toluene was removed by drying. Thus, a drug reservoir layer having a thickness of 10 $g/m^2$ was obtained. Subsequently, a patch 3 was obtained by stacking a polyethylene terephthalate film (support layer) on a surface of the drug reservoir layer on the side opposite from the release liner.

(Patch Production Example 4)

A patch 4 was obtained in the same manner as in Patch Production Example 3, except that the composition of the drug reservoir layer was as follows:

[Drug Reservoir Base Agent]
styrene-isoprene-styrene block copolymer (SIS): 70 parts by mass, and
[Drug]
rivastigmine (free form): 30 parts by mass.

(Patch Production Example 5)

A patch 5 was obtained in the same manner as in Patch Production Example 3, except that the composition of the drug reservoir layer was as follows:

[Drug Reservoir Base Agent]

SIS: 23.3 parts by mass,
tackifier: 23.3 parts by mass, and
liquid paraffin (LP): 23.3 parts by mass, and

[Drug]

rivastigmine (free form): 30 parts by mass.
Note that the tackifier used was an alicyclic saturated hydrocarbon resin (AP) (ARKONP100, manufactured by Arakawa Chemical Industries, Ltd.).

(Patch Production Example 6)

A patch 6 was obtained in the same manner as in Patch Production Example 3, except that the composition of the drug reservoir layer was as follows:

[Drug Reservoir Base Agent]

PIB: 23.3 parts by mass,
AP: 23.3 parts by mass, and
LP: 23.3 parts by mass, and

[Drug]

rivastigmine (free form): 30 parts by mass.

(Patch Production Example 7)

A patch 7 was obtained in the same manner as in Patch Production Example 1, except that an initial composition of the drug reservoir layer was as follows:

[Drug Reservoir Base Agent]

acrylic polymer 1: 93.32 parts by mass, and
sodium hydroxide: 0.88 parts by mass, and

[Drug]

tulobuterol hydrochloride: 5.80 parts by mass (5 parts by mass in terms of free form).
As the acrylic polymer 1, Duro-Tak 387-2516 was used with the acrylic polymer 1 being contained at the above-described parts by mass (hereinafter, the same shall apply). Note that tulobuterol was contained in the free form in the obtained drug reservoir layer.

(Patch Production Example 8)

A patch 8 was obtained in the same manner as in Patch Production Example 1, except that an initial composition of the drug reservoir layer was as follows:

[Drug Reservoir Base Agent]

acrylic polymer 1: 93.68 parts by mass, and
sodium hydroxide: 0.69 parts by mass, and

[Drug]

terbinafine hydrochloride: 5.63 parts by mass (5 parts by mass in terms of free form).
Note that terbinafine was contained in the free form in the obtained drug reservoir layer.

(Patch Production Example 9)

A patch 9 was obtained in the same manner as in Patch Production Example 1, except that an initial composition of the drug reservoir layer was as follows:

[Drug Reservoir Base Agent]

acrylic polymer 2: 93.68 parts by mass, and

sodium hydroxide: 0.69 parts by mass, and

[Drug]

terbinafine hydrochloride: 5.63 parts by mass (5 parts by mass in terms of free form).
As the acrylic polymer 2, Duro-Tak 387-2194 was used with the acrylic polymer 2 being contained at the above-described parts by mass (hereinafter, the same shall apply). Note that terbinafine was contained in the free form in the obtained drug reservoir layer.

(Patch Production Example 10)

A patch 10 was obtained in the same manner as in Patch Production Example 1, except that an initial composition of the drug reservoir layer was as follows:

[Drug Reservoir Base Agent]

acrylic polymer 1: 88.52 parts by mass, and
sodium hydroxide: 2.64 parts by mass, and

[Drug]

emedastine fumarate: 8.84 parts by mass (5 parts by mass in terms of free form).

Note that emedastine was contained in the free form in the obtained drug reservoir layer.

(Patch Production Example 11)

A patch 11 was obtained in the same manner as in Patch Production Example 1, except that an initial composition of the drug reservoir layer was as follows:

[Drug Reservoir Base Agent]

acrylic polymer 2: 88.52 parts by mass, and
sodium hydroxide: 2.64 parts by mass, and

[Drug]
emedastine fumarate: 8.84 parts by mass (5 parts by mass in terms of free form).

Note that emedastine was contained in the free form in the obtained drug reservoir layer.

(Cover Material Production Example 1)

First, a solution of an acrylic polymer 2 (adhesive base agent) in ethyl acetate (Duro-Tak 87-2194, acrylic polymer 2: 100 parts by mass) was used as an adhesive layer composition. The obtained adhesive layer composition was applied onto one surface of a polyethylene terephthalate film (release liner) having been subjected to a release treatment, and then ethyl acetate was removed by drying. Thus, an adhesive layer having a thickness of 50 g/m$^2$ was obtained. Subsequently, a cover material 1 was obtained by stacking a polyethylene terephthalate film (cover layer) on a surface of the adhesive layer on the side opposite from the release liner.

(Cover Material Production Example 2)

A cover material 2 was obtained in the same manner as in Cover Material Production Example 1, except that the composition of the adhesive base agent in the adhesive layer was as follows:

acrylic polymer 1: 100 parts by mass.

Note that, as the acrylic polymer 1, Duro-Tak 387-2516 was used with the acrylic polymer 1 being contained at 100 parts by mass.

(Cover Material Production Example 3)

First, an adhesive layer composition was obtained by stirring 90 parts by mass of PIB (adhesive base agent) in toluene, and adding, to this mixture, a solution of an acrylic polymer 1 (adhesive base agent) in ethyl acetate (Duro-Tak 387-2516, acrylic polymer 1: 10 parts by mass). The obtained adhesive layer composition was applied onto one surface of a polyethylene terephthalate film (release liner) having been subjected to a release treatment, and then toluene and ethyl acetate were removed by drying. Thus, an adhesive layer having a thickness of 50 g/m$^2$ was obtained. Subsequently, a cover material 3 was obtained by stacking a polyethylene terephthalate film (cover layer) on a surface of the adhesive layer on the side opposite from the release liner.

(Cover Material Production Example 4)

A cover material 4 was obtained in the same manner as in Cover Material Production Example 3, except that the composition of the adhesive base agent in the adhesive layer was as follows:

PIB: 80 parts by mass, and
acrylic polymer 1: 20 parts by mass.

Note that, as the acrylic polymer 1, Duro-Tak 387-2516 was used with the acrylic polymer 1 being contained at 20 parts by mass.

(Cover Material Production Example 5)

First, an adhesive layer composition was obtained by stirring 100 parts by mass of PIB (adhesive base agent) in toluene. The obtained adhesive layer composition was applied onto one surface of a polyethylene terephthalate film (release liner) having been subjected to a release treatment, and then toluene was removed by drying. Thus, an adhesive layer having a thickness of 50 g/m$^2$ was obtained. Subsequently, a cover material 5 was obtained by stacking a polyethylene terephthalate film (cover layer) on a surface of the adhesive layer on the side opposite from the release liner.

(Cover Material Production Example 6)

A cover material 6 was obtained in the same manner as in Cover Material Production Example 5, except that the composition of the adhesive base agent in the adhesive layer was as follows:

PIB: 33.3 parts by mass,
AP: 33.3 parts by mass, and
LP: 33.3 parts by mass.

(Cover Material Production Example 7)

A cover material 7 was obtained in the same manner as in Cover Material Production Example 5, except that the composition of the adhesive base agent in the adhesive layer was as follows:

PIB: 72 parts by mass,
AP: 8 parts by mass, and
LP: 20 parts by mass.

(Cover Material Production Example 8)

A cover material 8 was obtained in the same manner as in Cover Material Production Example 5, except that the composition of the adhesive base agent in the adhesive layer was as follows:

PIB: 81 parts by mass,

AP: 9 parts by mass, and
LP: 10 parts by mass.

(Cover Material Production Example 9)

A cover material 9 was obtained in the same manner as in Cover Material Production Example 5, except that the composition of the adhesive base agent in the adhesive layer was as follows:

PIB: 75 parts by mass, and
AP: 25 parts by mass.

(Cover Material Production Example 10)

A cover material 10 was obtained in the same manner as in Cover Material Production Example 5, except that the composition of the adhesive base agent in the adhesive layer was as follows:

PIB: 90 parts by mass, and
AP: 10 parts by mass.

(Cover Material Production Example 11)

A cover material 11 was obtained in the same manner as in Cover Material Production Example 5, except that the composition of the adhesive base agent in the adhesive layer was as follows:

PIB: 77.8 parts by mass, and
LP: 22.2 parts by mass.

(Cover Material Production Example 12)

A cover material 12 was obtained in the same manner as in Cover Material Production Example 5, except that the composition of the adhesive base agent in the adhesive layer was as follows:

PIB: 90 parts by mass, and
LP: 10 parts by mass.

(Cover Material Production Example 13)

A cover material 13 was obtained in the same manner as in Cover Material Production Example 5, except that the composition of the adhesive base agent in the adhesive layer was as follows:

SIS: 33.3 parts by mass,
AP: 33.3 parts by mass, and
LP: 33.3 parts by mass.

(Cover Material Production Example 14)

A cover material 14 was obtained in the same manner as in Cover Material Production Example 5, except that the composition of the adhesive base agent in the adhesive layer was as follows:

SIS: 25 parts by mass,
AP: 25 parts by mass, and
LP: 50 parts by mass.

(Cover Material Production Example 15)

A cover material 15 was obtained in the same manner as in Cover Material Production Example 5, except that the composition of the adhesive base agent in the adhesive layer was as follows:

SIS: 25 parts by mass,
AP: 50 parts by mass, and
LP: 25 parts by mass.

(Cover Material Production Example 16)

A cover material 16 was obtained in the same manner as in Cover Material Production Example 5, except that the composition of the adhesive base agent in the adhesive layer was as follows:

SIS: 10 parts by mass, and
PIB: 90 parts by mass.

(Cover Material Production Example 17)

A cover material 17 was obtained in the same manner as in Cover Material Production Example 5, except that the composition of the adhesive base agent in the adhesive layer was as follows:

silicone-based adhesive agent (PSA4102, manufactured by Dow Corning Corporation): 100 parts by mass.

(Example 1)

First, the patch 2 was cut into a 5 cm$^2$ piece, and the cover material 5 was cut into a 15 cm$^2$ piece. Then, the release liner of the cover material was detached. Subsequently, as shown in Fig. 2, the adhesive layer of the cover material and the support layer of the patch were laminated to each other with the patch being arranged at a center portion of the adhesive layer, and further end portions of the adhesive layer not in contact with the support layer were brought into contact with side surfaces of the drug reservoir layer. Thus, a cover material-equipped patch was obtained. In addition, a polyethylene terephthalate film (release liner) having been subjected to a release treatment was stacked on end portions of the adhesive layer not in contact with the support layer or the side surfaces of the drug reservoir layer as shown in Fig. 2. Thus, a cover material-equipped patch was obtained. The obtained cover material-equipped patch was measured for the amount of the drug transferred. Table 1 shown below shows the combination of the cover material and the patch in the obtained cover material-equipped patch, and also shows the drug transfer concentration determined by the measurement for the amount of the drug transferred. In addition, Table 1 also shows the solubility parameter (SPb) of the drug reservoir base agent, the solubility parameter (SPc) of the adhesive base agent, the difference (SPa-SPb) between the solubility parameter (SPa) of the drug and SPb, the difference (SPa-SPc) between SPa and SPc, and the difference (SPb-SPc) between SPb and SPc of the obtained cover material-equipped patch. Note that, in Table 1, each of the solubility parameters (SPa, SPb, and SPc) was a value determined from the composition of the compounds used for the corresponding one of the drug and the base agents by using the above-described Fedors' calculation formula. In addition, SPb and SPc were each an SP value of a component (or a mixture of components in the case of the mixture of components) selected from adhesive agents (acrylic polymer 1, acrylic polymer 2, PIB, SIS, and silicone-based adhesive agent in the cases of the above-described Examples) and additives (AP and LP in the cases of the above-described Example). Each SPa was the SP value of the drug alone, namely, the SP value of rivastigmine in the free form was 10.4, the SP value of tulobuterol in the free form was 11.61, the SP value of terbinafine in the free form was 9.77, and the SP value of emedastine in the free form was 10.47.

(Examples 2 to 15 and Comparative Example(Comp. Ex.)s 1 to 28)

Cover material-equipped patches were each obtained in the same manner as in Example 1, except that the combination of the patch and the cover material was changed to the corresponding one of the combinations shown in Tables 1 to 3. Each of the obtained cover material-equipped patches was measured for the amount of the drug transferred. Tables 1 to 3 show the combinations of the cover material and the patch in the cover material-equipped patches, and also show the drug transfer concentrations determined by the measurement for the amount of the drug transferred. Tables 1 to 3 also show SPb, SPc, the difference (SPa-SPb) between SPa and SPb, the difference (SPa-SPc) between SPa and SPc, and the difference (SPb-SPc) between SPb and SPc of each of the obtained cover material-equipped patches.

(Example 16)

First, a drug reservoir layer composition prepared in the same manner as in Patch Production Example 1 was applied onto one surface of a polyethylene terephthalate film (release liner) having been subjected to a release treatment, and then ethyl acetate was removed by drying. Thus, a drug reservoir layer having a thickness of 200 g/m$^2$ was obtained. Subsequently, a polyethyleneterephthalate film (support layer) not subjected to any release treatment was stacked on a surface of the drug reservoir layer on the side opposite from the release liner. Meanwhile, 13.5 parts by mass of SIS, 13.5 parts by mass of PIB, 27 parts by mass of LP, 36 parts by mass of AP, and 10 parts by mass of Eudragid L100 (manufactured by Evonik Industries AG) in terms of solid content were stirred in toluene, and were applied onto one surface of another polyethylene terephthalate film (release liner) having been subjected to a release treatment . Then, toluene was removed by drying. Thus, a patch adhesive layer having a thickness of 150 g/m$^2$ was obtained. Subsequently, the release liner was removed from the drug reservoir layer, and the drug reservoir layer and the patch adhesive layer were laminated to each other. The laminate was cut into a size of 5 cm$^2$. In this manner, a patch 12 (multilayer type patch) was obtained in which the support layer, the drug reservoir layer, the patch adhesive layer, and the release liner were stacked in this order. Subsequently, a cover material 5 obtained in the same manner as in Cover Material Production Example 5 was cut into a 15 cm$^2$ piece. Then, a cover material-equipped patch was obtained in the same manner as in Example 1, except that this piece of the cover material 5 was used in combination with the patch 12. Note that the difference (SPa-SPb) between SPa and SPb, the difference (SPa-SPc) between SPa and SPc, and the difference (SPb-SPc) between SPb and SPc of the obtained cover material-equipped patch satisfied the conditions represented by the above-described (1) to (3), and measurement for the amount of the drug transferred showed that the drug transfer concentration was 1.22%.

[Table 1]

| | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Example 1 | Patch 2 | 8.99 | Cover material 5 | 7.70 | 1.41 | 2.70 | 1.29 | 1.41 |
| Example 2 | Patch 1 | 9.72 | Cover material 16 | 7.74 | 0.68 | 2.66 | 1.98 | 1.73 |
| Example 3 | Patch 1 | 9.72 | Cover material 10 | 7.85 | 0.68 | 2.55 | 1.87 | 2.03 |
| Example 4 | Patch 1 | 9.72 | Cover material 12 | 7.79 | 0.68 | 2.61 | 1.93 | 2.19 |
| Example 5 | Patch 1 | 9.72 | Cover material 5 | 7.70 | 0.68 | 2.70 | 2.02 | 2.24 |
| Example 6 | Patch 1 | 9.72 | Cover material 9 | 8.09 | 0.68 | 2.31 | 1.63 | 2.34 |
| Example 7 | Patch 1 | 9.72 | Cover material 8 | 7.92 | 0.68 | 2.48 | 1.80 | 2.38 |
| Example 8 | Patch 1 | 9.72 | Cover material 11 | 7.89 | 0.68 | 2.51 | 1.83 | 2.64 |
| Example 9 | Patch 1 | 9.72 | Cover material 3 | 7.90 | 0.68 | 2.50 | 1.82 | 2.77 |

(continued)

|  | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Example 10 | Patch 1 | 9.72 | Cover material 4 | 8.10 | 0.68 | 2.30 | 1.62 | 2.80 |
| Example 11 | Patch 1 | 9.72 | Cover material 7 | 8.00 | 0.68 | 2.40 | 1.72 | 2.81 |
| Comp. Ex. 1 | Patch 4 | 8.13 | Cover material 5 | 7.70 | 2.27 | 2.70 | 0.43 | 3.04 |
| Comp. Ex. 2 | Patch 5 | 8.64 | Cover material 5 | 7.70 | 1.76 | 2.70 | 0.94 | 3.18 |
| Comp. Ex. 3 | Patch 3 | 7.70 | Cover material 5 | 7.70 | 2.70 | 2.70 | 0.00 | 3.29 |
| Comp. Ex. 4 | Patch 1 | 9.72 | Cover material 6 | 8.50 | 0.68 | 1.90 | 1.22 | 3.52 |
| Comp. Ex. 5 | Patch 6 | 8.50 | Cover material 5 | 7.70 | 1.90 | 2.70 | 0.80 | 3.82 |
| Comp. Ex. 6 | Patch 1 | 9.72 | Cover material 15 | 8.79 | 0.68 | 1.61 | 0.93 | 4.21 |
| Comp. Ex. 7 | Patch 1 | 9.72 | Cover material 13 | 8.64 | 0.68 | 1.76 | 1.08 | 4.86 |

[Table 2]

|  | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Comp. Ex. 8 | Patch 3 | 7.70 | Cover material 1 | 8.99 | 2.70 | 1.41 | -1.29 | 5.34 |
| Comp. Ex. 9 | Patch 1 | 9.72 | Cover material 14 | 8.62 | 0.68 | 1.78 | 1.10 | 5.68 |
| Comp. Ex. 10 | Patch 6 | 8.50 | Cover material 1 | 8.99 | 1.90 | 1.41 | -0.49 | 5.76 |
| Comp. Ex. 11 | Patch 2 | 8.99 | Cover material 1 | 8.99 | 1.41 | 1.41 | 0.00 | 6.72 |

(continued)

| | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Comp. Ex. 12 | Patch 3 | 7.70 | Cover material 2 | 9.72 | 2.70 | 0.68 | -2.02 | 7.33 |
| Comp. Ex. 13 | Patch 2 | 8.99 | Cover material 2 | 9.72 | 1.41 | 0.68 | -0.73 | 7.87 |
| Comp. Ex. 14 | Patch 2 | 8.99 | Cover material 13 | 8.64 | 1.41 | 1.76 | 0.35 | 9.29 |
| Comp. Ex. 15 | Patch 3 | 7.70 | Cover material 13 | 8.64 | 2.70 | 1.76 | -0.94 | 10.50 |
| Comp. Ex. 16 | Patch 1 | 9.72 | Cover material 1 | 8.99 | 0.68 | 1.41 | 0.73 | 11.50 |
| Comp. Ex. 17 | Patch 1 | 9.72 | Cover material 2 | 9.72 | 0.68 | 0.68 | 0.00 | 13.25 |
| Comp. Ex. 18 | Patch 5 | 8.64 | Cover material 1 | 8.99 | 1.76 | 1.41 | -0.35 | 14.97 |
| Comp. Ex. 19 | Patch 6 | 8.50 | Cover material 13 | 8.64 | 1.90 | 1.76 | -0.14 | 15.06 |
| Comp. Ex. 20 | Patch 5 | 8.64 | Cover material 2 | 9.72 | 1.76 | 0.68 | -1.08 | 15.79 |
| Comp. Ex. 21 | Patch 4 | 8.13 | Cover material 1 | 8.99 | 2.27 | 1.41 | -0.86 | 16.57 |
| Comp. Ex. 22 | Patch 4 | 8.13 | Cover material 2 | 9.72 | 2.27 | 0.68 | -1.59 | 17.98 |
| Comp. Ex. 23 | Patch 4 | 8.13 | Cover material 13 | 8.64 | 2.27 | 1.76 | -0.51 | 18.00 |
| Comp. Ex. 24 | Patch 6 | 8.50 | Cover material 2 | 9.72 | 1.90 | 0.68 | -1.22 | 19.59 |
| Comp. Ex. 25 | Patch 5 | 8.64 | Cover material 13 | 8.64 | 1.76 | 1.76 | 0.00 | 19.70 |
| Comp. Ex. 26 | Patch 1 | 9.72 | Cover material 17 | 7.40 | 0.68 | 3.00 | 2.32 | 21.66 |

(continued)

| | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Comp. Ex. 27 | Patch 2 | 8.99 | Cover material 17 | 7.40 | 1.41 | 3.00 | 1.59 | 38.46 |

[Table 3]

| | Patch | SP value of drug reservoir layer (SPb) | Cover material | SP value of adhesive layer (SPc) | SPa-SPb | SPa-SPc | SPb-SPc | Drug transfer concentration [%] |
|---|---|---|---|---|---|---|---|---|
| Example 12 | Patch 8 | 9.72 | Cover material 5 | 7.70 | 0.05 | 2.07 | 2.02 | 1.28 |
| Example 13 | Patch 9 | 8.99 | Cover material 5 | 7.70 | 0.78 | 2.07 | 1.29 | 0.97 |
| Example 14 | Patch 10 | 9.72 | Cover material 5 | 7.70 | 0.75 | 2.77 | 2.02 | 0.28 |
| Example 15 | Patch 11 | 8.99 | Cover material 5 | 7.70 | 1.48 | 2.77 | 1.29 | 0.00 |
| Comp. Ex. 28 | Patch 7 | 9.72 | Cover material 5 | 7.70 | 1.89 | 3.91 | 2.02 | 3.46 |

As is apparent from the results shown above, it was found that the transfer of the drug from the drug reservoir layer to the adhesive layer, i.e., from the patch to the cover material was sufficiently reduced in each of the cover material-equipped patches of the present invention in which the relationship among the solubility parameter (SPa) of the drug, the solubility parameter (SPb) of the drug reservoir base agent, and the solubility parameter (SPc) of the adhesive base agent satisfied the specific conditions.

[Industrial Applicability]

As described above, according to the present invention, it is possible to provide a cover material-equipped patch and a cover material-equipped patch kit which can be attached for a long period, and with which the transfer of the drug from the patch to the cover material is sufficiently reduced.

In addition, according to the present invention, the transfer of the drug from the patch to the cover material is sufficiently reduced, and hence the release of the drug from the cover material to the skin is reduced. Hence, it is possible to provide a cover material-equipped patch and a cover material-equipped patch kit excellent in sustained-release. Moreover, the cover material-equipped patch and the cover material-equipped patch kit make it possible to keep the concentration of the drug in the blood constant for a long period, because the temporal increases and the subsequent decreases in the amount of the drug released and the amount of the drug permeating through the skin are suppressed, and the drug is gradually and continuously released from the patch.

[Reference Signs List]

1:      cover material-equipped patch
10:     patch
20:     cover material
30:     release liner
101:    support layer
102:    drug reservoir layer
201:    cover layer
202:    adhesive layer

**Claims**

1. A cover material-equipped patch, comprising:

a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa - SPb| \leq 1.5 \cdots (1),$$

$$2.0 \leq (SPa - SPc) \leq 2.8 \cdots (2),$$

and

$$1.0 \leq (SPb - SPc) \leq 2.8 \cdots (3).$$

2. The cover material-equipped patch according to claim 1, wherein
the formula (1) is the following formula:

$$0 \leq (SPa - SPb) \leq 1.5 \cdots (1').$$

3. The cover material-equipped patch according to claim 1 or 2, wherein
the drug is at least one selected from the group consisting of rivastigmine, terbinafine, emedastine, and pharmaceutically acceptable salts thereof.

4. The cover material-equipped patch according to any one of claims 1 to 3, wherein
the drug reservoir base agent contains an acrylic adhesive agent.

5. The cover material-equipped patch according to claim 4, wherein
the acrylic adhesive agent is a polymer obtained by polymerization of at least one selected from the group consisting of (meth)acrylic acid, 2-ethylhexyl (meth)acrylate, methyl (meth)acrylate, butyl (meth)acrylate, and hydroxyethyl (meth)acrylate.

6. The cover material-equipped patch according to any one of claims 1 to 5, wherein
the adhesive base agent contains a rubber-based adhesive agent.

**7.** The cover material-equipped patch according to claim 6, wherein
the rubber-based adhesive agent is at least one selected from the group consisting of styrene-isoprene-styrene block copolymer, styrene-butadiene-styrene block copolymer, styrene-ethylene-butylene-styrene block copolymer, natural rubber, polyisobutylene, and polyisoprene.

**8.** The cover material-equipped patch according to any one of claims 1 to 7, wherein
an amount of the drug transferred from the drug reservoir layer to the adhesive layer during storage at a temperature of 60°C for 2 weeks is 3% by mass or less of the drug contained in the drug reservoir layer before the storage.

**9.** A cover material-equipped patch kit comprising:

a patch; and
a cover material, wherein
the patch comprises a support layer and a drug reservoir layer which is stacked on one surface of the support layer and which contains a drug and a drug reservoir base agent,
the cover material comprises a cover layer and an adhesive layer which is stacked on one surface of the cover layer and which contains an adhesive base agent,
the patch and the cover material are to be arranged in such a manner that the adhesive layer is stacked on another surface of the support layer, and
a solubility parameter (SPa) of the drug, a solubility parameter (SPb) of the drug reservoir base agent, and a solubility parameter (SPc) of the adhesive base agent simultaneously satisfy conditions represented by the following formulae (1) to (3):

$$0 \leq |SPa - SPb| \leq 1.5 \cdots (1),$$

$$2.0 \leq (SPa - SPc) \leq 2.8 \cdots (2),$$

and

$$1.0 \leq (SPb - SPc) \leq 2.8 \cdots (3).$$

Fig.1

Fig.2

**INTERNATIONAL SEARCH REPORT**

| International application No. |
| --- |
| PCT/JP2014/082727 |

| A. CLASSIFICATION OF SUBJECT MATTER |
| --- |
| *A61K9/70*(2006.01)i, *A61K31/27*(2006.01)i, *A61K47/32*(2006.01)i, *A61K47/34*(2006.01)i, *A61P25/28*(2006.01)i |

According to International Patent Classification (IPC) or to both national classification and IPC

| B. FIELDS SEARCHED |
| --- |

Minimum documentation searched (classification system followed by classification symbols)
A61K9/70, A61K31/27, A61K47/32, A61K47/34, A61P25/28

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | WO 2011/074636 A1 (Takeshi GOTO), 23 June 2011 (23.06.2011), entire text & US 2012/0323190 A1 & EP 2514415 A1 & AU 2010331243 A & CA 2784712 A & CN 102834093 A & KR 10-2013-0000370 A & TW 201127422 A | 1-9 |
| A | WO 2005/072669 A1 (Hisamitsu Pharmaceutical Co., Inc.), 11 August 2005 (11.08.2005), entire text & US 2007/0190123 A1 | 1-9 |

| [×] Further documents are listed in the continuation of Box C. | [ ] See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 30 January 2015 (30.01.15) | 10 February 2015 (10.02.15) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (July 2009)

**INTERNATIONAL SEARCH REPORT**

| International application No. |
|---|
| PCT/JP2014/082727 |

C (Continuation).  DOCUMENTS CONSIDERED TO BE RELEVANT

| Category* | Citation of document, with indication, where appropriate, of the relevant passages | Relevant to claim No. |
|---|---|---|
| A | JP 7-247221 A  (Sekisui Chemical Co., Ltd.),<br>26 September 1995 (26.09.1995),<br>entire text<br>(Family: none) | 1-9 |
| A | JP 9-315957 A  (Hisamitsu Pharmaceutical Co., Inc.),<br>09 December 1997 (09.12.1997),<br>entire text<br>(Family: none) | 1-9 |
| A | JP 2013-237691 A  (Noven Pharmaceuticals, Inc.),<br>28 November 2013 (28.11.2013),<br>entire text<br>& US 2005/0169977 A1     & EP 1682061 A1<br>& CA 2542778 A             & KR 10-2006-0114338 A | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009517468 A
- JP 5093545 B
- WO 2011074636 A
- WO 200507266 A
- JP SHO61158924 B
- WO 2005072669 A

**Non-patent literature cited in the description**

- **FEDORS R. F.** A method for estimating both the solubility parameters and molar volumes of liquids. *Poly. Eng. Sci.,* 1974, vol. 14, 147-154